# EUROPEAN PATENT SPECIFICATION

(11) **EP 2 566 958 B1**
(45) Date of publication and mention of the grant of the patent: **30.12.2015**
(21) Application number: 11716419.4
(22) Date of filing: 28.04.2011
(51) Int. Cl.: C12N 7/06, A61K 39/12, A61K 39/145

(54) **METHOD FOR INACTIVATING INFLUENZA VIRUS**
VERFAHREN ZUR INAKTIVIERUNG DES INFLUENZA-VIRUS
PROCÉDÉ POUR L'INACTIVATION DU VIRUS DE LA GRIPPE

(30) Priority: 03.05.2010 US 330443 P
(43) Date of publication of application: 13.03.2013
(73) Proprietor: GlaxoSmithKline Biologicals S.A., 1330 Rixensart (BE)
(72) Inventor: ANDRE, Bruno, Rene, B-1330 Rixensart (BE); CHAMPLUVIER, Benoit, Paul, Suzanne, B-1330 Rixensart (BE)
(74) Representative: Van Den Hazel, Hendrik Bart
(86) International application number: PCT/EP2011/056762
(87) International publication number: WO 2011/138229

(56) References cited:
- WO-A1-2009/029695
- US-A- 5 698 432
- US-A1- 2006 270 017
- GOLDSTEIN M A ET AL: "Effect of formalin, beta-propiolactone, merthiolate, and ultraviolet light upon influenza virus infectivity chicken cell agglutination, hemagglutination, and antigenicity.", APPLIED MICROBIOLOGY FEB 1970 LNKD- PUBMED:5437304, vol. 19, no. 2, February 1970 (1970-02), pages 290-294, XP002649006, ISSN: 0003-6919
- DATABASE EMBASE [Online] ELSEVIER SCIENCE PUBLISHERS, AMSTERDAM, NL; 1955, HARTMAN F W ET AL: "Four - Year study concerning the inactivation of viruses in blood and plasma", XP002649008, Database accession no. EMB-0007342311 & HARTMAN F W ET AL: "Four - Year study concerning the inactivation of viruses in blood and plasma", GASTROENTEROLOGY (NEW YORK, N.Y. 1943) 1955, vol. 28, no. 2, 1955, pages 244-256, ISSN: 0016-5085
- LAVENDER J F: "Purified rabies vaccine (suckling rat brain origin).", APPLIED MICROBIOLOGY JUN 1970 LNKD- PUBMED:5456012, vol. 19, no. 6, June 1970 (1970-06), pages 923-927, XP002649007, ISSN: 0003-6919

## Description

### TECHNICAL FIELD

The present invention relates to a method for inactivating influenza viruses and contaminating adventitious viruses produced in cell culture. The method of the invention is simple and easy to implement. In particular, it can be suitably inserted during any virus purification process.

### TECHNICAL BACKGROUND

Due to the vast number of diseases caused by viruses, virology has been an intensively studied field. There has always been the demand to produce viruses efficiently in order to isolate and purify viral proteins, to generate vaccines, to prepare analytical tools, or to provide viruses for laboratory studies.

Recently, cell culture-based technologies as an alternative to the traditional egg-derived production systems have been developed.

Cell culture systems appear as a suitable alternative mode of vaccine preparation in particular, simpler, flexible, consistent, allowing to improve possibilities of up-scaling vaccine production capacities and thus to reach large quantities of virus, if needed, in particular, in case of a pandemic threat or a terrorist attack.

Irrespective of the production mode, the virus, in particular, if it is intended to be used for vaccination, needs to be safe and therefore infectivity needs to be reduced or eliminated. This may require the virus to be attenuated. Alternatively this may require the virus to be purified and inactivated. Whilst it is necessary to irreversibly reduce, as much as possible, the infectivity of the virus, its immunogenicity should however be preserved. It is also essential that vaccines be free of adventitious agents, as the host for producing the virus, such as eggs or cell cultures, may have been contaminated with viruses during their culture. Different inactivating agents are known in the art, such as detergents, or chemical agents, such as formaldehyde and beta-propiolactone (BPL). However, problems may be associated with respect to the use of said agents: (i) very often, the use of these agents does not allow a complete inactivation of the virus suspension to be treated, as a significant residual infectivity has been frequently reported; (ii) detergents have to be used with caution as they may have a detrimental impact on the structure of viral proteins, which may be essential for the virus immunogenicity, their detergent-induced denaturation, leading, eventually, to an impaired vaccine efficacy, due to immunogenicity loss; (iii) chemical agents, such as BPL and formaldehyde, need to be manipulated cautiously and require specific equipments to manipulate them, as they are often harmful products and present health risks to people manipulating them; (iv) formaldehyde, in particular, a standard way of inactivating viruses, requires usually long incubation times for inactivating virus, from 1 to several days, and it is also known to be responsible for aggregating proteins, said aggregation which may negatively affect subsequent steps required for virus purification; (iv) detergents and chemical agents need to be eliminated from the virus suspension after their use, adding thus complexity to virus purification processes. The implementation of these techniques of inactivating an orthomyxovirus may further lead to significant residual infectivity still being present.

UV light is also known in the art as a means for inactivating infectious viruses. For example, WO 2008/039494 discloses a device and methods of inactivating infectious virus, such as influenza virus, and inactivating potential adventitious agents, such as adventitious viruses, with ultraviolet (UV) light. However, WO 2008/039494 also teaches that UV treatment did not provide significant inactivation of the adventitious viruses which were actually tested.

Therefore, a need remains for providing alternative influenza virus inactivation methods, simple to implement, and allowing to achieve a complete virus inactivation, as residual infectivity is not acceptable or vaccines safety, which method should also prove useful for inactivating possible adventitious viruses, which may contaminate the fluid comprising the virus of interest.

### SUMMARY OF THE INVENTION

The method according to the present invention provides a method for inactivating an influenza virus which overcomes the above-mentioned drawbacks. In particular, the method of the invention allows to achieve a high level of virus inactivation, while preserving virus antigenicity.

In a first aspect of the present invention, there is provided a method for inactivating an influenza virus propagated in cell culture and inactivating contaminating adventitious viruses comprising at least the following steps:
(a) treating an influenza virus-containing fluid with beta-propiolactone, and
(b) irradiating the influenza virus-containing fluid with UV light.

In a second aspect of the present invention, there is provided a method for the preparation of a vaccine comprising an influenza virus propagated in cell culture comprising the following steps:
(a) treating an influenza virus-containing and contaminating adventitious viruses containing fluid with beta-propiolactone,
(b) irradiating the influenza virus-containing and contaminating adventitious viruses containing fluid with UV light, and
(c) formulating the beta-propiolactone-treated and UV-irradiated influenza virus into a vaccine.

The present disclosure also describes a virus obtainable according to the method of the invention.

The present disclosure further describes an immunogenic composition comprising a virus obtained according to the present invention admixed with a suitable pharmaceutical carrier.

The present disclosure further describes an immunogenic composition comprising a virus obtained according to the present invention for use in medicine. In particular, it is described an immunogenic composition for use in preventing or treating orthomyxoviruses associated infections.

### DETAILED DESCRIPTION

The present disclosure relates to an improved method of inactivating viruses that can be applied to both small and large scale virus production. The method described therein is applicable, in particular, to egg-derived virus and cell culture-derived virus. The method involves the implementation of at least one inactivation step, with at least two distinct inactivating agents. More specifically, the method described therein relies on the combination of a physical treatment, such as UV irradiation, and a chemical treatment, in particular a treatment involving an alkylation step, such as with BPL, in order to inactivate the viruses. Optionally, said method further comprises a detergent-based splitting step.

The inventors found out that the specific combination of inactivation means as disclosed herein, combining a physical treatment and a chemical treatment, allowed achieving a more complete virus inactivation, *i.e.* elimination of the residual infectivity that may be associated with the prior art treatments. They surprisingly observed that this combination, in addition to providing a complete virus inactivation, does not affect the integrity of the viral proteins produced, nor their antigenicity. The resulting virus, whether further purified or not, is suitable for use in an immunogenic composition, such as a vaccine composition.

The claimed method has one or more of the following advantages: (i) it optimises the virus accessibility to the treatment, whether the virus is aggregated or not, whether it is trapped within cell debris, whether or not the conformation, or the environmental conditions, are optimal for a virus inactivation step; (ii) it reduces the risk that a virus fraction may escape the inactivation when only one step of inactivation is performed thereby achieving a more complete inactivation of the virus; (iii) it reduces the risk of incomplete inactivation associated with the resistance that the virus may exhibit against a specific treatment, whether this resistance is linked to the nature of the virus or to the experimental/environmental conditions of the treatment. This may be the case when for instance, only a fraction of viruses within a virus suspension may be inactivated by a chemical agent at a given concentration but, for safety reasons or immunogenicity reasons, in particular, it may be impossible to further increase the concentration of the inactivating agent. An additional advantage of the claimed method is that implementing UV irradiation may allow decreasing the amount of the chemical agent which is used, as compared to the situation where inactivation is performed only by using the chemical agent; (iv) it reduces the time required for inactivating the produced virus, as compared to prior art treatments, in particular, formaldehyde-based treatment.

Any chemical agent known to have an inactivating effect on virus can be used in the method described in the present disclosure, such as for instance an alkylating agent (e.g. BPL). BPL is used in the method of the invention, as, in addition to its inactivating capability, it presents the advantage of degrading nucleic acids, such as DNA. This additional ability is of a particular importance if the virus produced in cell culture as described herein is to be included in a vaccine. Indeed, for safety reasons, the residual DNA content, which originates from the host cell used for propagating the virus, must be kept as low as possible and its size, as small as possible. BPL is a mono-alkylating agent which reacts with various biological molecules, in particular, it modifies the nucleic bases of the viral genome and blocks its replication. It can be rapidly inactivated by heating at 37°C for 2 hours since it is completely hydrolyzed to non-toxic products beta-hydroxypropionic acid and an isomer of lactate. The method for inactivating an influenza virus of the present invention comprises at least one step of treating a virus-containing fluid with BPL (BPL treatment) and one step of irradiating the virus-containing fluid with UV light (UV irradiation). In one embodiment, BPL treatment and UV irradiation are performed sequentially, in any order. For instance, BPL treatment may precede UV irradiation, or it may follow it. Alternatively, in the method described in the present disclosure, BPL treatment and UV irradiation steps may be implemented simultaneously. This presents the additional advantage of simplifying and shortening the process by saving an extra-step. Also, BPL and UV irradiation may be separated by other purification steps, or they may be implemented in a successive manner, in the sense that BPL treatment is immediately followed by UV treatment or UV treatment is immediately followed by BPL treatment, with no further purification step in between,

The terms "virus-containing fluid", such as an orthomyxovirus-containing fluid, and "fluid comprising a virus" are synonymous and are to be understood as any liquid preparation comprising a virus, irrespective of its purification status. The fluid may have not been purified at all. For instance, the fluid may be the cell culture supernatant collected after cells were infected with a virus and the virus was replicated and released into the medium. Alternatively, the fluid may have been partially purified. For example, the virus-containing fluid may have been pre-clarified, by filtration or by centrifugation, before being subject to inactivation by a BPL treatment and a UV irradiation.

Virus inactivation may be achieved with less than 1 % BPL. In one embodiment of the invention, BPL is used at a concentration ranging from 0.01% to 0.1 %, or from 0.03% to 0.8%, or is 0.05%. BPL is appropriately added to a buffered solution and the pH of the solution is maintained between 6 and 10. As BPL activity is known to be particularly sensitive to pH, the pH of the buffer solution is maintained between 6 and 9, suitably between 7 and 8, and more suitably between 7.4 and 8. In one aspect of the disclosure, BPL may be added to a phosphate 66 mM - Citrate 125 mM pH 7.4 buffer solution. BPL is active at a temperature ranging from 4°C to room temperature. In one aspect of the disclosure, the incubation temperature of BPL may be 4°C. In a distinct aspect of the disclosure, the incubation temperature of BPL may be room temperature. In the sense of the present disclosure, room temperature is to be understood as a temperature comprised between about 20°C and about 24°C. The incubation time may vary between one hour and a few days. In one aspect of the disclosure, BPL may be added overnight. In the sense of the present disclosure, an overnight incubation means an incubation time of at least 8 hours, possibly ranging from 12-16 hours. In one aspect of the disclosure, BPL may be added at a concentration of 0.05% at 4°C overnight. In a distinct aspect of the disclosure, BPL may be added at a concentration of 0.05% at room temperature overnight. After BPL is added and left for the appropriate period of time at room temperature, the BPL-treated virus suspension may be stored at 4°C for a few days, such as for example three days, before further purifying the virus suspension if needed.

Suitable UV irradiation occurs through a type C light, *i.e*. light with a wavelength ranging from 100 to 280, suitably from 200 and 270 nm, and more suitably 254 nm which is the maximal absorption area by nucleic acids of treated viruses. During UV inactivation, the excitation energy of the UV wavelength radiation disrupts the covalent bonds of the purine and pyrimidin bases, resulting in damage to target virus as well as adventitious agents and bacterial bioburden. UV doses or fluency may range, in particular, from 50 to 500 J/m². The present invention contemplates any UV dose leading to virus inactivation. According to one embodiment of the method of the present invention, the UV fluency ranges from 50 to 500 J/m², from 100 to 400 J/m² or is 200 Joules/m² or is 100 Joules/m². In one aspect of the disclosure, the UV fluency may be 60 Joules/m². The available commercial devices for UV irradiation allow to handle small to very large volumes of fluid. As an illustration only, the following device may be cited : UVIVATEC^{™} (from Bayer).

BPL conditions and UV fluencies will be determined in order to achieve the optimal conditions for virus inactivation, according to the amount and to the type of virus which needs to be inactivated. The skilled person may use any known in the art assays for assessing viral inactivation, such as measuring the Tissue Culture Infectious dose (TCID₅₀/ml), which represents the amount of a virus capable of infecting 50% of cells. In this assay, a cell culture is scored as either infected or not, allowing to determine a virus titration, as the measure of the virus infectivity. A series of successive dilutions of the infectious samples to be tested are performed and part of each dilution is used for inoculating suitable infectable cells. After incubating the cells for a few days, so that the virus, if infectious, can replicate, the presence of the virus may be detected by two reading methods known to the skilled person, the analysis of the cytopathic effect (CPE) in cells and/or the hemagglutination assay with chicken red blood cells performed on the culture supernatant. The viral titer is then calculated according to the Reed and Muench method (Reed, L.J. and Muench, H., 1938, The American Journal of Hygiene 27: 493-497). If willing to preserve antigenicity and immunogenicity, the skilled person will adapt the virus inactivation conditions so as to not alter the viral proteins conformation, or structure. In parallel to the inactivation tests, he will monitor the integrity of the virus, or of specific proteins thereof, by any known-in-the art-technique. Techniques which may be cited as illustrative examples are any protein detection technique, such as Western-blot analysis with specific antibodies, or Threshold assays. In the particular case of influenza virus, the content of the protein HA, known to be immunogenic, can be specifically monitored by the SRD (single radial immunodiffusion assay) assay, which is a technique familiar to a person skilled in the art (J.M. Wood et al.: An improved single radial immunodiffusion technique for the assay of influenza haemagglutinin antigen: adaptation for potency determination of inactivated whole virus and subunit vaccines. J. Biol. Stand. 5 (1977) 237-247; J. M. Wood et al., International collaborative study of single radial diffusion and immunoelectrophoresis techniques for the assay of haemagglutinin antigen of influenza virus. J. Biol. Stand. 9 (1981) 317-330)). As the SRD assay relies on the formation of complexes antibody/antigen, said assay, when assessing the HA level from an influenza virus suspension, allows to analyse if the inactivation treatment of that suspension negatively impacts the virus antigenicity.

In addition to inactivate influenza virus, the inactivation method of the present invention also allows inactivating potential adventitious viruses that may contaminate the influenza virus-containing fluid. In particular, these viruses may have been present in the host producing the influenza virus, or they may have contaminated the influenza virus-containing fluid along its purification, at any step. Regulatory authorities, for instance in the vaccination field, are becoming stricter and impose that vaccines be tested against numerous potential contaminating viruses. By way of examples of such viruses, may PPV (Porcine Parvovirus), MuLV (Murine-Leukemia virus), PRV (Porcine Pseudorabies virus) and HAV (Hepatitis A virus) be cited. Parvoviruses are small non-enveloped DNA viruses which infect lots of animal species. They are extremely resistant, in particular, to chemical treatments. The method according to the invention allows to inactivate a wide range of adventitious viruses, such as, but not limited to, MuLV, PRV, HAV and PPV. In particular, the method of the present invention is used for inactivating at least one of the contaminating MuLV, PRV, HAV and PPV, or any combination thereof from influenza virus produced in cell culture. In one aspect of the disclosure, the method described therein may be used for inactivating all of them. The elimination of adventitious viruses, when relating to contaminant viruses, is often called viral clearance. Accordingly, in the sense of the present disclosure, "viral clearance" is to be understood as the capacity to eliminate adventitious viruses which may contaminate the suspension of the virus of interest. Viral clearance can be monitored and assessed by any suitable method allowing to detect the presence of a given virus. For example, the assessment may be direct, *i.e.* the presence of the endogenous virus, if present as a contaminant, may be directly detected from the virus suspension to be tested, by PCR, for example, using primers specific for the genome of the contaminant virus of interest. However, for sensitivity reasons, as contaminants viruses are expected to be only present at very low level, if any, it may be more suitable to use an indirect assessment relying, for instance, on the addition of exogenous virus. In that case, the virus suspension to be inactivated is spiked with the adventitious virus of interest, *i.e.* with a known amount of the adventitious virus for which the viral clearance needs to be determined. The spiking occurs before implementing any virus inactivation step, so as to have a sufficient amount of viruses to serve as a reference before inactivation and possibly purification. Then, the inactivation step of interest is carried out on the spiked virus suspension, for example, the inactivating method as described therein. Virus titration is determined before and after the implementation of the inactivation method on the spiked virus suspension, so that the infectivity of the adventitious virus used for spiking can be evaluated and, thus, the effectiveness of the inactivation method can be assessed. Purification steps may be associated with the inactivation steps, and the combination thereof may, thus, be also assessed on the infectivity of an adventitious virus of interest.

The virus obtained using the method of the present invention can be used for any purpose; including, for instance, purification of viral proteins, analytical assays, infection of host cells, diagnostic purposes or therapeutic or prophylactic uses such as vaccination and clinical administration. In particular, the virus obtained using the method of the present invention may be suitable for use in an immunogenic composition such as in a vaccine.

According to one aspect of the disclosure, the method described therein can be applied to other viruses, in particular to any virus which is capable of infecting cells and using them for its replication, including, but not limited to, adenoviruses, hepadnaviruses, herpes viruses, orthomyxoviruses, papovaviruses, paramyxoviruses, picornaviruses, poxviruses, reoviruses and retroviruses. In particular, the method described therein is suitable for enveloped viruses, such as myxoviruses.

Viruses or viral antigens may consequently be derived from an Orthomyxovirus, such as influenza virus. Orthomyxovirus antigens may be selected from one or more of the viral proteins, including hemagglutinin (HA), neuraminidase (NA), nucleoprotein (NP), matrix protein (M1), membrane protein (M2), one or more of the transcriptase (PB1, PB2 and PA). Particularly suitable antigens include HA and NA, the two surface glycoproteins which determine the antigenic specificity of the Influenza subtypes.

Influenza virus is selected from the group consisting of human influenza virus, avian influenza virus, equine influenza virus, porcine (e.g. swine) influenza virus, feline influenza virus. Influenza virus is more particularly selected from strains A, B and C, preferably from strains A and B.

Influenza virus or antigens thereof may be derived from interpandemic (annual or seasonal) influenza strains. Alternatively, influenza virus or antigens thereof may be derived from strains with the potential to cause a pandemic outbreak (*i.e*., influenza strains with new hemaggultinin compared to hemagglutinin in currently circulating strains, or influenza strains which are pathogenic in avian subjects and have the potential to be transmitted horizontally in the human population, or influenza strains which are pathogenic to humans). Depending on the particular season and on the nature of the antigen included in the vaccine, the influenza virus or antigens thereof may be derived from one or more of the following hemagglutinin subtypes: H1, H2, H3, H4, H5, H6, H7, H8, H9, H10, H11, H12, H13, H14, H15 or H16. Preferably, the influenza virus or antigens thereof are from H1, H2, H3, H5, H7 or H9 subtypes.

The cells which may be used in the method described therein can in principle be any desired cell type of cells which can be cultured in cell culture and which can support virus replication. They can be both adherently growing cells or cells growing in suspension. They can be either primary cells or continuous cell lines. Genetically stable cell lines are preferred.

Mammalian cells are particularly suitable, for example, human, hamster, cattle, monkey or dog cells.

A number of mammalian cell lines are known in the art and include PER.C6, HEK cells, human embryonic kidney cells (293 cells), HeLa cells, CHO cells, Vero cells, and MDCK cells.

Suitable monkey cells are, for example, African green monkey cells, such as kidney cells as in Vero cell line. Suitable dog cells are, for example, kidney cells as in MDCK cell line.

Suitable mammalian cell lines for growing influenza virus include MDCK cells, Vero cells, or PER.C6 cells. These cell lines are all widely available, for instance, from the American Type Cell Culture (ATCC) collection.

In one aspect of the disclosure, the method described therein may use MDCK cells. The original MDCK cell line is available from the ATCC as CCL-34, but derivatives of this cell line may also be used, such as the MDCK cells adapted to growth in suspension (WO 1997/37000).

Alternatively, cell lines for use in the invention may be derived from avian sources, such as chicken, duck, goose, quail or pheasant. Avian cell lines may be derived from a variety of developmental stages including embryonic, chick and adult. In particular, cell lines may be derived from the embryonic cells, such as embryonic fibroblasts, germ cells, or individual organs, including neuronal, brain, retina, kidney, liver, heart, muscle, or extraembryonic tissues and membranes protecting the embryo. Chicken embryo fibroblasts (CEF) may be used. Examples of avian cell lines include avian embryonic stem cells (WO01/85938) and duck retina cells (WO05/042728). In particular, the EB66^{®} cell line derived from duck embryonic stem cells is contemplated in the present invention (WO 2008/129058). Other suitable avian embryonic stem cells include the EBx cell line derived from chicken embryonic stem cells, EB45, EB14 and EB14-074 (WO2006/108846). This EBx cell line presents the advantage of being a genetically stable cell line whose establishment has been produced naturally and did not require any genetic, chemical or viral modification. These avian cells are particularly suitable for growing influenza viruses.

According to a particular embodiment, the method of the invention uses EB66^{®} cells.

Cell culture conditions (temperature, cell density, pH value, etc ...) are variable over a very wide range owing to the suitability of the cells employed and can be adapted to the requirements of particular virus growth conditions details. It is within the skilled person's capabilities to determine the appropriate culture conditions, as cell culture is extensively documented in the art (see, for example, Tissue Culture, Academic Press, Kruse and Paterson, editors (1973), and R.I. Freshney, Culture of animal cells: A manual of basic technique, fourth edition (Wiley-Liss Inc., 2000, ISBN 0-471-34889-9).

In one aspect of the disclosure, host cells used in the method described in the present disclosure may be cultured in serum-free and/or protein-free media. A "serum-free medium" (SFM) means a cell culture medium ready to use that does not require serum addition allowing cell survival and cell growth. This medium may not necessarily be chemically defined and may contain hydrolyzates of various origin, from plant for instance. Such serum-free medium present the advantage that contamination with viruses, mycoplasma or unknown infectious agents can be ruled out. "Protein-free" is understood to mean cultures in which multiplication of the cells occurs with exclusion of proteins, growth factors, other protein additives and non-serum proteins, but can optionally include proteins such as trypsin or other proteases that may be necessary for viral growth. The cells growing in such culture naturally contain protein themselves.

Serum-free media are commercially available from numerous sources, for instance, VP SFM (Invitrogen Ref 11681-020), Opti-Pro (Invitrogen, Ref 12309-019), or EX-CELL (JHR Bioscience).

Cells may be grown in various ways, for instance, in suspension, or adhering to surfaces, including growth on microcarriers, or combinations thereof. Culturing can be done in dishes, flasks, roller bottles, or in bioreactors, using batch, fed-batch, semi-continuous or continuous systems, such as perfusion systems. Typically, cells are scaled-up from a master or working cell bank vial through various sizes of flasks or roller bottles and finally to bioreactors. In one aspect of the disclosure, the cells employed in the method described therein may be cultured on microcarrier beads in a serum-free medium in a stirred-bioreactor and the culture medium is provided by perfusion.

In a distinct aspect of the disclosure, cells may be cultured in suspension in a batch mode, in particular, EB66^{®} cells.

Prior to infection with the virus, cells may be cultured around 37°C, more suitably at 36.5°C, at a pH ranging from 6.7 to 7.8, suitably around 6.8 to 7.5, and more suitably around 7.2

According to the present disclosure, the production of cell culture-based viruses includes generally the steps of inoculating the cultured cells with the viral strain to be produced and cultivating the infected cells for a desired period of time so as to allow virus replication.

In order to produce large quantities of cell-produced viruses, it is preferred to inoculate cells with the desired virus strain once cells have reached a high density. Usually, the inoculation is performed when the cell density is at least around 1.5 x 10⁶ cells/ml, suitably, around 3 x 10⁸ cells/ml, more suitably, around 5 x 10⁸ cells/ml, even more suitably 7 x 10⁸ cells/ml, or even higher. The optimal cell density for obtaining the highest virus production may vary according to the cell type used for the virus propagation.

The inoculation can be carried out at an MOI (Multiplicity Of Infection) of about 10⁻¹ to 10⁻⁷, suitably about 10⁻² to 10⁻⁶, and more suitably, about 10⁻⁵.

The temperature and pH conditions for virus infection may vary. Temperature may range from 32°C to 39°C depending on the virus type. For Influenza virus production, cell culture infection may vary depending on the strain which is produced. Influenza virus infection may be suitably performed at a temperature ranging from 32°C to 35°C, suitably at 33°C. In one aspect of the disclosure, the virus infection may occur at 33°C. In a distinct aspect of the disclosure, the virus infection may take place at 35°C. Proteases, typically trypsin, may be added to the cell culture depending on the virus strain, to allow viral replication. The protease can be added at any suitable stage during the culture. Tryspin is preferably of non-animal origin, that is to say the protease is not purified from an animal source. It is suitably recombinantly produced in a micro-organism, such as bacterial, yeast or plant. Suitable examples of recombinant trypsin are Trypzean, a recombinant trypsin produced in corn (Prodigen, 101 Gateway Blvd, Suite 100 College Station, Texas 77845. Manufacturer code : TRY), or TrpLE (Invitrogen) which is a trypsin-like enzyme expressed in fungus (WO2004/020612).

Once infected, cells may release into the culture medium newly formed virus particles, due to spontaneous lysis of host cells, also called passive lysis. Therefore, in one aspect of the disclosure, cell-based viral harvest may be provided any time after virus inoculation by collecting the cell culture medium or supernatant. In one aspect of the disclosure, the cell culture medium may be collected by perfusion. This mode of harvesting is particularly suitable when it is desired to harvest cell-derived virus at different time points after virus inoculation, and pooling the different harvests, if needed.

Alternatively, after virus infection, cell-based virus may be harvested by employing external factor to lyse host cells, also called active lysis. However, contrary to the previous one, such a harvesting mode requires that the cell-based viral harvest be collected at a single time point, as actively lysing the cells will immediately terminate the cell culture.

Methods that can be used for active cell lysis are known. Useful methods in this respect are for example, freeze-thaw, solid shear, hypertonic and/or hypotonic lysis, liquid shear, high pressure extrusion, detergent lysis, or any combination thereof.

In one aspect of the disclosure, cell-based viral harvest may be provided any time after virus inoculation by collecting the cell culture medium or supernatant, lysing the inoculated cells or both.

Before harvesting, cell infection may last for 2 to 10 days. In one aspect of the disclosure, culture supernatants from days 3, 4 and 5 post-inoculation may be harvested and pooled for further downstream processing (virus isolation). In a distinct aspect of the disclosure, cell culture supernatant may be collected from day 5 post-inoculation. The optimal time to harvest the cell-produced virus is usually based on the determination of the infection peak. For example, the CPE (CytoPathic Effect) is measured by monitoring the morphological changes occurring in host cells after virus inoculation, including cell rounding, disorientation, swelling or shrinking, death, detachment from the surface. The detection of a specific viral antigen may also be monitored by standard techniques of protein detection, such as a Western-blot analysis. Harvest can then be collected when the desired detection level is achieved. In the particular case of influenza virus, the content of HA may be monitored any time post-inoculation of the cells with the virus, by the SRD assay (Wood, JM, et al. (1977). J. Biol. Standard. 5, 237-247), which is a technique familiar to a person skilled in the art. Additionally, the SRD assay may also be used for determining the optimal cell density range required to obtain an optimized virus yield.

In the context of the present disclosure, the cell culture phase is to be understood as encompassing any step preceding the virus harvesting step, while the virus purification phase is to be understood as encompassing any step following said harvesting step. For instance, the virus purification phase of cell culture-based viruses may include a number of different filtration, concentration and/or other separation steps such as ultrafiltration, ultracentrifugation (including gradient ultracentrifugation), chromatography (such as ion exchange chromatography) and adsorption steps in a variety of combinations. The method for inactivating viruses of the present invention may suitably be associated with any virus purification process by implementing the chemical inactivation and the UV irradiation step at any appropriate step.

In one embodiment, the inactivation method of the invention comprises at least one further step selected from the group of: clarification, whether by centrifugation or filtration, ultrafiltration/diafiltration, nucleic acids degradation, ultracentrifugation, in particular sucrose gradient ultracentrifugation and chromatography, or any combination thereof.

A combination of BPL treatment and UV irradiation may suitably be implemented, in any order, after any of the above step. By way of example, a BPL treatment may be implemented early in a virus purification process, in particular, after clarification of the viral harvest obtained by collecting the virus-containing cell culture medium. This treatment may be followed immediately by UV irradiation. Alternatively, BPL treatment and UV irradiation may be separated by other purification steps. Implementing a first step of virus inactivation as early in the process, *i.e*. on the clarified harvest, provides, in particular, a safety advantage as far as operating people are concerned. Indeed, as the virus from the fluid which needs to be purified is inactivated, and thus non-infectious, early in the process, operating people are protected from a possible virus infection. However, such an early stage for inactivation also provides the drawback of having to deal with large volumes. Therefore, the cost associated with the inactivation step, in particular, the BPL step, might be quite significant, due to the large volume of the virus suspension to be inactivated. Accordingly, the inventors also tested whether inactivating the virus later in the purification process, such as for example, after ultafiltration and concentration of the clarified harvest and/or after a sucrose gradient ultracentrifugation step would still provide good inactivation results. They observed that implementing BPL and UV inactivation steps on purified virus suspension did provide inactivation results, at least as good as when inactivation is implemented before purifying the virus, and possibly better.

In one aspect of the disclosure, the influenza virus-containing cell culture medium produced in mammals MDCK cells or avian EB66® cells, may be successively, clarified by filtration or by centrifugation, inactivated by a BPL treatment, concentrated by ultrafiltration and subject to UV irradiation.

Alternatively, the BPL treatment may be implemented after the clarified virus harvest has been concentrated, for instance by ultrafiltration. Concentrating the virus harvest first allows to use less amount of BPL, which not only represents a cost advantage, as indicated above, but also allows manipulators to handle lower amounts of BPL. UV irradiation may occur immediately after the BPL treatment, or later, after additional purifications steps implemented after said BPL treatment.

After inactivation, the virus obtained may be further purified. Accordingly, the influenza virus-containing cell culture medium, which has been subject, successively, to clarification by centrifugation and/or filtration, inactivation by a BPL treatment, concentration by ultrafiltration and to UV irradiation may be further subject to a sucrose gradient ultracentrifugation.

Alternatively, the inactivation steps according to the present invention may occur later in the purification process. For example, the virus harvest may pre-clarified by centrifugation, clarified by micro-filtration, subject to ultrafiltration and concentration, and subject to a sucrose gradient ultracentrifugation. The whole virus-containing fractions collected from the sucrose gradient ultracentrifugation are then subject to inactivation. In one aspect of the disclosure, a UV irradiation in a range of 50 to 200 J/m² may be first performed on the fractions, and then, BPL may be added, in particular, BPL 0.05%, to the irradiated collected virus fractions. BPL may be left overnight, in particular between 12 to 16 hours at room temperature. In a distinct aspect of the disclosure, BPL may be added first directly to the collected virus fractions, in particular, BPL 0.05%, and left at least overnight at room temperature. The next day, the BPL-treated virus suspension may be subject to UV irradiation, such as for example UV 100 J/m². While residual sucrose present within the collected virus fractions might have impact the effectiveness of BPL at inactivating the virus, due to its known stabilizing effect on virus, the inventors surprisingly observed that residual sucrose, if any, within a virus-containing fluid to be inactivated did not affect the inactivating effect produced by BPL. Therefore, the inactivation method of the present invention relying on the combination of BPL treatment and UV irradiation, has a broad application in that inactivation steps can be implemented at different stages of the production and the purification of influenza virus, including immediately after a sucrose gradient ultracentrifugation step.

The method for inactivating viruses as described therein may also be associated with an additional step for degrading residual contaminating DNA from the host which produced the virus. For instance, a DNA degradation step with a nuclease, such as, but not limited to, Benzonase^{™}, may be implemented in addition to the combination of a chemical inactivation and UV irradiation.

The method according to the present invention further contemplates a possible splitting step. For example, it may be possible to combine a purification step, such as sucrose gradient ultracentrifugation, with a virus splitting step. In particular, a splitting agent may be added to the sucrose gradient. This is particularly suitable, when it is desired to minimize the total number of steps of the method of the invention, as it allows, within a single operation, to both purify and split the virus. Hence, in one aspect of the disclosure, when at least one sucrose gradient ultracentrifugation is implemented, the sucrose gradient additionally may comprise a splitting agent.

Alternatively, the virus splitting step of the method of the present invention may be performed in batch.

Methods of splitting viruses, such as influenza viruses, are well known in the art (WO02/28422). Splitting of the virus may be carried out by disrupting or fragmenting whole virus whether infectious (wild-type or attenuated) or non-infectious (inactivated) with a disrupting concentration of a splitting agent. Splitting agents generally include agents capable of breaking up and dissolving lipid membranes. Traditionally, split influenza virus was produced using a solvent/detergent treatment, such as tri-n-butyl phosphate, or diethylether in combination with Tween^{™} (known as "Tween-ether" splitting) and this process is still used in some production facilities. Other splitting agents now employed include detergents or proteolytic enzymes or bile salts, for example sodium deoxycholate. Detergents that can be used as splitting agents include cationic detergents e.g. cetyl trimethyl ammonium bromide (CTAB), other ionic detergents, e.g. sodium lauryl sulphate (SLS), taurodeoxycholate, or non-ionic detergents such as Tween^{™} or Triton X-100, or combination of any two or more detergents.

In one aspect of the disclosure, the splitting agent may be deoxycholate. In another aspect of the disclosure, the splitting agent may be Triton X-100. Triton X-100 may be suitably used at a concentration ranging from 0.5% to 3%, in particular, 1% to 2%. In a further aspect of the disclosure, the method described therein may use a combination of Triton X-100 and sodium lauryl sulfate as splitting agents. In one aspect of the disclosure, after a BPL treatment and a UV irradiation, in any order, the virus-containing fluid, such as an influenza virus-containing fluid may be splitted in batch with Triton X-100, in particular Triton X-100 2%.

The splitting process may be carried out as a batch, continuous or semi-continuous process. When implemented in batch, the split virus may require an additional step of purification to eliminate the detergent, such as a chromatography step.

In one aspect of the disclosure, the viral harvest may be clarified, by centrifugation and/or filtration, optionally concentrated by ultrafiltration, purified by a sucrose gradient ultracentrifugation step, inactivated by a BPL treatment and a UV irradiation step, sequentially, in any order, and the inactivated virus fluid may be split in batch by adding a detergent, suitably Triton X-100.

Immunogenic compositions of the present disclosure, including vaccines, can optionally contain the additives customary for vaccines, in particular substances which increase the immune response elicited in a patient who receives the composition, *i.e*. so-called adjuvants.

In one aspect of the disclosure, immunogenic compositions may comprise a virus or a viral antigen thereof obtainable according to the present invention admixed with a suitable pharmaceutical carrier. In one aspect of the disclosure, they may further comprise an adjuvant.

Adjuvant compositions may comprise an oil in water emulsion which comprise a metabolisable oil and an emulsifying agent. In order for any oil in water composition to be suitable for human administration, the oil phase of the emulsion system has to comprise a metabolisable oil. The meaning of the term metabolisable oil is well known in the art. Metabolisable can be defined as 'being capable of being transformed by metabolism' (Dorland's Illustrated Medical Dictionary, W.B. Sanders Company, 25th edition (1974)). The oil may be any vegetable oil, fish oil, animal oil or synthetic oil, which is not toxic to the recipient and is capable of being transformed by metabolism. Nuts, seeds, and grains are common sources of vegetable oils. Synthetic oils are also part of this disclosure and can include commercially available oils such as NEOBEE® and others.

A particularly suitable metabolisable oil is squalene. Squalene (2,6,10,15,19,23-Hexamethyl-2,6,10,14,18,22-tetracosahexaene) is an unsaturated oil which is found in large quantities in shark-liver oil, and in lower quantities in olive oil, wheat germ oil, rice bran oil, and yeast, and is a particularly preferred oil. Squalene is a metabolisable oil by virtue of the fact that it is an intermediate in the biosynthesis of cholesterol (Merck index, 10th Edition, entry no.8619). In a further aspect of the disclosure, the metabolisable oil may be present in the immunogenic composition in an amount of 0.5% to 10% (v/v) of the total volume of the composition.

The oil-in-water emulsion may further comprise an emulsifying agent. The emulsifying agent may suitably be polyoxyethylene sorbitan monooleate. Further, said emulsifying agent may be suitably present in the vaccine or immunogenic composition 0.125 to 4% (v/v) of the total volume of the composition.

The oil-in-water emulsion of the present disclosure may optionally comprise a tocol. Tocols are well known in the art and are described in EP0382271. Suitably may be a tocol is alpha-tocopherol or a derivative thereof such as alpha-tocopherol succinate (also known as vitamin E succinate). Said tocol may be suitably present in the adjuvant composition in an amount 0.25% to 10% (v/v) of the total volume of the immunogenic composition.

The method of producing oil-in-water emulsions is well known to the person skilled in the art. Commonly, the method comprises mixing the oil phase (optionally comprising a tocol) with a surfactant such as a PBS/TWEEN80™ solution, followed by homogenisation using a homogenizer, it would be clear to a man skilled in the art that a method comprising passing the mixture twice through a syringe needle would be suitable for homogenising small volumes of liquid. Equally, the emulsification process in microfluidiser (M110S Microfluidics machine, maximum of 50 passes, for a period of 2 minutes at maximum pressure input of 6 bar (output pressure of about 850 bar)) could be adapted by the man skilled in the art to produce smaller or larger volumes of emulsion. The adaptation could be achieved by routine experimentation comprising the measurement of the resultant emulsion until a preparation was achieved with oil droplets of the required diameter.

In an oil-in-water emulsion, the oil and emulsifier are in an aqueous carrier. The aqueous carrier may be, for example, phosphate buffered saline.

In particular, the oil-in-water emulsion systems of the present disclosure may have a small oil droplet size in the sub-micron range. Suitably the droplet sizes may be in the range 120 to 750 nm, more particularly sizes from 120 to 600 nm in diameter. Even more particularly, the oil-in water emulsion may contain oil droplets of which at least 70% by intensity are less than 500 nm in diameter, more particular at least 80% by intensity are less than 300 nm in diameter, more particular at least 90% by intensity are in the range of 120 to 200 nm in diameter.

The oil droplet size, *i.e*. diameter, according to the present disclosure is given by intensity. There are several ways of determining the diameter of the oil droplet size by intensity. Intensity is measured by use of a sizing instrument, suitably by dynamic light scattering such as the Malvern Zetasizer 4000 or suitably the Malvern Zetasizer 3000HS. A detailed procedure is given in Example II.2. A first possibility is to determine the z average diameter ZAD by dynamic light scattering (PCS-Photon correlation spectroscopy); this method additionally give the polydispersity index (PDI), and both the ZAD and PDI are calculated with the cumulants algorithm. These values do not require the knowledge of the particle refractive index. A second mean is to calculate the diameter of the oil droplet by determining the whole particle size distribution by another algorithm, either the Contin, or NNLS, or the automatic "Malvern" one (the default algorithm provided for by the sizing instrument). Most of the time, as the particle refractive index of a complex composition is unknown, only the intensity distribution is taken into consideration, and if necessary the intensity mean originating from this distribution.

The adjuvant compositions may further comprise a Toll like receptor (TLR) 4 agonist. By "TLR4 agonist" it is meant a component which is capable of causing a signalling response through a TLR4 signalling pathway, either as a direct ligand or indirectly through generation of endogenous or exogenous ligand (Sabroe et al, JI 2003 p1630-5). The TLR 4 may be a lipid A derivative, particularly monophosphoryl lipid A or more particularly 3 Deacylated monophoshoryl lipid A (3 D - MPL).

3D-MPL is available under the trademark MPL® by GlaxoSmithKline Biologicals North America and primarily promotes CD4+ T cell responses with an IFN-g (Th1) phenotype. It can be produced according to the methods disclosed in GB 2 220 211 A. Chemically it is a mixture of 3-deacylated monophosphoryl lipid A with 3, 4, 5 or 6 acylated chains. In particular, in the adjuvant compositions of the present disclosure small particle 3 D- MPL is used. Small particle 3 D -MPL has a particle size such that it may be sterile-filtered through a 0.22µm filter. Such preparations are described in International Patent Application No. WO 94/21292. Synthetic derivatives of lipid A are known and thought to be TLR 4 agonists including, but not limited to:
**OM174** (2-deoxy-6-o-[2-deoxy-2-[(R)-3-dodecanoyloxytetra-decanoylamino]-4-o-phosphono-β-D-glucopyranosyl]-2-[(R)-3-hydroxytetradecanoylamino]-α-D-glucopyranosyldihydrogenphosphate), (WO 95/14026)
**OM 294** DP (3S, 9 R) -3--[(R)-dodecanoyloxytetradecanoylamino]-4-oxo-5-aza-9(R)-[(R)-3-hydroxytetradecanoylamino]decan-1,10-diol,1,10-bis(dihydrogenophosphate) (WO99/64301 and WO 00/0462)
**OM 197** MP-Ac DP (3S-, 9R) -3-[(R) -dodecanoyloxytetradecanoylamino]-4-oxo-5-aza-9-[(R)-3-hydroxytetradecanoylamino]decan-1,10-diol,1 -dihydrogenophosphate 10-(6-aminohexanoate) (WO 01/46127)

Other TLR4 ligands which may be used are alkyl Glucosaminide phosphates (AGPs) such as those disclosed in WO9850399 or US6303347 (processes for preparation of AGPs are also disclosed), or pharmaceutically acceptable salts of AGPs as disclosed in US6764840. Some AGPs are TLR4 agonists, and some are TLR4 antagonists. Both are thought to be useful as adjuvants. In addition, further suitable TLR-4 agonists are disclosed in US2003/0153532 and US2205/0164988.

The invention is particularly suitable for preparing influenza virus immunogenic compositions, including vaccines. Various forms of influenza virus are currently available. They are generally based either on live virus or inactivated virus. Inactivated vaccines may be based on whole virions, spilt virions or purified surface antigens (including HA). Influenza antigens can also be presented in the form of virosomes (nucleic acid-free viral-like liposomal particle).

Influenza virus strains for use in vaccines change from season to season. In the current interpandemic period, vaccines typically include two influenza A strains and one influenza B strain. Trivalent vaccines are typical, but higher valence, such as quadrivalence, is also described in the present disclosure. The present disclosure may also use HA from pandemic strains (*i.e.* strains to which the vaccine recipient and the general human population are immunologically naïve), and influenza vaccines for pandemic strains may be monovalent or may be based on a normal trivalent vaccine supplemented by a pandemic strain.

Compositions of the present disclosure may include antigen(s) from one or more influenza virus strains, including influenza A virus and/or influenza B virus. In particular, a trivalent vaccine including antigens from two influenza A virus strains and one influenza B virus strain is described in the present disclosure.

The compositions of the disclosure are not restricted to monovalent compositions, *i.e.* including only one strain type, *i.e.* only seasonal strains or only pandemic strains. The disclosure also relates to multivalent compositions comprising a combination of seasonal strains and/or of pandemic strains. In particular, a quadrivalent composition, which may be adjuvanted, comprising three seasonal strains and one pandemic strain are also described in the present disclosure. Other compositions described in the present disclosure are a trivalent composition comprising two A strains and one B strain, such as H1N1, H3N2 and B strains, and a quadrivalent composition comprising two A strains and two B strains of a different lineage, such as H1N1, H3N2, B/Victoria and B/Yamagata.

HA is the main immunogen in current inactivated influenza vaccines, and vaccine doses are standardized by reference to HA levels, typically measured by SRD. Existing vaccines typically contain about 15 µg of HA per strain, although lower doses can be used, *e.g.* for children, or in pandemic situations, or when using an adjuvant. Fractional doses such as a half (*i.e.* 7.5 µg HA per strain) or a quarter have been used, as have higher doses, in particular, 3x or 9x doses. Thus immunogenic compositions of the present disclosure may include between 0.1 and 150 µg of HA per influenza strain, particularly, between 0.1 and 50 µg, *e.g.* 0.1-20 µg, 0.1-15 µg, 0.1-10 µg, 0.1-7.5 µg, 0.5-5 µg, etc. Particular doses include about 15, about 10, about 7.5, and about 5 µg per strain.

Once an influenza virus has been purified for a particular strain, it may be combined with viruses from other strains to make a trivalent vaccine, for example, as described above. It is more suitable to treat each strain separately and to mix monovalent bulks to give a final multivalent mixture, rather than to mix viruses and degrade DNA and purify it from a multivalent mixture.

The invention will be further described by reference to the following, non-limiting, examples.

### Example 1: BPL-induced inactivation of influenza virus produced on MDCK cells (NCP124 - New Caledonia A strain, NCP127 - New Caledonia A strain, JP125 - Jiangsu B strain, JP128 - Jiangsu B strain, NCP134 - New Caledonia A strain, and JP129 - Jiangsu B strain)

The MDCK adherent cells were grown on microcarriers in a perfusion culture mode at 36.5°C. After the growth phase, once the appropriate cell density was reached (ranging from 4.5 x 10⁶ cells/ml to 7.5 x 10⁶ cells/ml), cells were inoculated with different strains of Influenza virus (Multiplicity of Infection of 1 x 10⁻⁵) in a perfusion mode and the temperature was switched to 33°C. The virus was harvested by perfusion a few days later. The perfusion harvests were pooled and the complete virus harvest was:
a) clarified on a filtration train composed of three different depth filters with the following nominal porosities: 5 µm - 0.5 µm - 0.2 µm. In the experiment NCP127, the viral harvest was added with Tween 80 at a final concentration of 0.02% before clarification.
b) the clarified harvests were, then, concentrated 10-fold (NCP124), 20-fold (JP128), or 30-fold (NCP127, JP125, NCP134 and JP129) by ultrafiltration with a 750 kD hollow fiber membrane, so as to get a final volume of around 2 liters, diafiltrated against 5 volumes of PBS containing 125 mM citrate and 0.01 % Triton X-100 and against 4 volumes of 10 mM Tris, 2 mM MgCl₂, 0.1 µM CaCl₂, 0.01 % Triton X-100,
c) the retentates were removed from the ultrafiltration system and warmed up to 37°C in a water bath. DNA degradation was performed by adding Benzonase^{™} (Merck) to the retentates at a final concentration of 100 Units/ml (NCP124), 200 Units/ml (JP125, NCP134 and JP129) or 135 Units/ml (NCP127 and JP128) and the mixture was incubated 1 hour at 37°C.
d) In the experiments JP128 and JP129 only, the Benzonase^{™}-treated retentate was homogenized by high pressure homogenization at 1000 bars.
e) the ultrafiltration retentates (whether homogenized or not) were, then, subject to a sucrose gradient (0-55%) ultracentrifugation, wherein virus and contaminants migrate into the gradient until reaching their respective density. Once all the retentate was loaded onto the gradient, a banding time of 60 minutes allowed most of the virus to reach its density within the gradient. The viral particles were concentrated within a few fractions. The product fractions are in PBS pH 7.4 containing 125 mM citrate and sucrose. The purified whole virion was pooled from the percentage of sucrose ranging from approximately 26 to 51%. This range has been determined on the basis of profiles from SDS-PAGE and from Western Blot analysis using anti-HA and anti-MDCK antibodies. The whole virion pooled fractions were stored at a temperature ranging from 4°C to 8°C.
f) a second sucrose gradient (5-55%) ultracentrifugation is performed in order to further purify the virus, while simultaneously splitting it. 2% Triton X-100 - 0.5 mM alpha-tocopheryl hydrogen succinate (NCP127, JP125, JP128, NCP134 and JP129) or 1.5% Triton X-100 - 1% Sodium Lauryl Sarcosinate - 0.5 mM alpha-tocopheryl hydrogen succinate (NCP124) was added to the sucrose layers to achieve a detergent micelles barrier. The whole virus entering this detergent barrier was split. Virus fragments containing the viral membrane proteins hemagglutinin (HA) and neuraminidase (NA) migrated to the micelles density. The remaining virions, some of the host cell protein contaminants and DNA migrated to higher sucrose concentration fractions which were not pooled with the viral proteins. The viral proteins present in the fractions ranging from approximately 13 to 55% sucrose were pooled. This range has been determined on the basis of profiles from SDS-PAGE and from Western blot analysis using anti-HA and anti-MDCK antibodies. The fractions pool containing the viral proteins were in PBS pH 7.4. This pool was then assayed for the total protein content and diluted to 250 µg protein/ml with PBS containing 0.01% Tween 80 - 0.3% Triton X-100 - alpha-tocopheryl hydrogen succinate 0.1 mM pH 7.4.

At least one step of virus inactivation with BPL was implemented during the above-described process, as follows:
- In the experiment NCP124, BPL was added after clarification of step a) at varying concentrations: 0.02, 0.04, 0.05, 0.06 and 0.1% and incubated overnight at 4°C.
- In the experiment JP125, BPL was added after the first sucrose gradient ultracentrifugation of step d) to the collected and pooled whole virions-containing fractions at a concentration of 0.1% and incubated overnight at 4°C.
- In the experiment NCP127, BPL was added after the retentate obtained at step b) was incubated with Benzonase^{™} at the step c) at varying concentrations: 0.02, 0.04, 0.05, 0.06, 0.08 and 0.1% and incubated overnight at 4°C.
- In the experiment JP128, BPL was added in the same conditions as JP125, except that varying concentrations were used: 0.05, 0.04, 0.03, 0.02 and 0.1%.
- In the experiment JP129, BPL was added in the same conditions as NCP127, except that the used concentration were: 0.03, 0.04 and 0.05%
- In the experiment NCP134, BPL was added in the same conditions as NCP127, except that used concentrations were 0.05% and 0.1 % and the incubation was overnight at room temperature (RT).

Virus inactivation was assessed by measuring the viral titration through the TCID₅₀ assay (Tissue-Culture-Infectious-Dose). At the end of the overnight incubation, a sample from every BPL conditions within each experiment was collected to test its infectiousness in order to evaluate the efficacy of BPL inactivation. A series of successive dilutions of the samples to be tested are performed. 50 µl of each dilution is inoculated in 10 replicates into a 96 wells microplate containing MDCK cells, 8 dilutions being inoculated for each sample to be tested. Plates are then incubated for 5-7 days at 35°C, so that the virus, if infectious, can replicate in cells. The presence of infectious virus in cells is detected by monitoring the Cytopathic effect (CPE) on the cells by microscopy. A suspension of infectious virus is used as a positive control to demonstrate cellular susceptibility and non-inoculated cultures are used as negative control. The number of wells where CPE is detected is scored for each dilution as infected cells and the viral titer is calculated according to Reed and Muench method (Reed, L.J. and Muench, H., 1938, The American Journal of Hygiene 27: 493-497). Results obtained are presented in Table 1 and expressed as log TCID₅₀/ml. The controls where no BPL (0) was added corresponds to the titration value assessed in a sample collected from the indicated virus harvest before it underwent any purification step.

**Table 1 - Effect of BPL on Flu virus titration**

| | % BPL | Titration log TCID₅₀/ml |
|---|---|---|
| NCP124 (4°C) | 0 | 8.93 |
| | 0.02 | 4.47 |
| | 0.04 | ≤ 1.8 |
| | 0.05 | ≤ 1.8 |
| | 0.06 | ≤ 1.8 |
| | 0.1 | ≤ 1.8 |
| JP125 | 0 | 11.3 |
| (4°C) | 0.1 | ≤ 1.8 |
| NCP127 (4°C) | 0 | 8.87 |
| | 0.05 | ≤ 1.8 |
| | 0.02 | 3.75 |
| | 0.04 | ≤ 1.6 |
| | 0.06 | ≤ 1.8 |
| | 0.08 | ≤ 1.8 |
| | 0.1 | ≤ 1.8 |
| JP128 (4°C) | 0 | 10.13 |
| | 0.05 | 4.8 |
| | 0.04 | 4.8 |
| | 0.03 | 6.93 |
| | 0.02 | 7.93 |
| | 0.01 | 8.13 |
| JP129 (4°C) | 0 | 9.98 |
| | 0.03 | 3.86 |
| | 0.04 | 5.8 |
| | 0.05 | 4.8 |
| NCP134 (RT) | 0 | 9.02 |
| | 0.05 | ≤ 1.8 |
| | 0.1 | ≤ 1.8 |

| | | |
|---|---|---|
| RT: Room Temperature 1.8 is the limit of quantitation of the assay | | |

### - Results-Conclusions

Table 1 indicates that BPL, when added to a virus fluid, is effective at inactivating viruses, as compared to the controls where no BPL was added. Most of the BPL concentrations used resulted in a viral titer equal or below the limit of quantitation of the assay and allow to achieve a viral titration reduction of about 8 logs, as compared to the controls where no BPL was added. The inactivating effect of BPL is observed irrespective of the incubation temperature (4°C or RT). A residual infectivity was observed in certain experiments, as some viral titers range from about 4 to about 8, and the virus titer reduction varies between 2 and 6 logs.

### Example 2: UV-induced inactivation of influenza virus produced on MDCK cells (JP128 - Jiangsu B strain)

The experimental cell culture conditions of JP128 were as described in Example 1. The viral harvest was clarified, as described in step a) of Example 1. After clarification, the viral clarified harvest has been subject to varying doses of UV: 200, 300, and 400. UV irradiation was carried out with UVivatec^{™} Lab device from Bayer Technical Services comprising a UV-C 254 nm low-pressure mercury lamp and according to the manufacturer's recommendations. The UV dose or fluency is a function of the lamp intensity and of the optical density (OD) of the sample to be irradiated measured at 254 nm. The value of OD at 254 nm, as well as the value of the desired fluency, are inserted into a Master Calculation sheet provided by Bayer in order to calculate the flow rate required for achieving said fluency. Table 2 indicates the parameters used for the UV irradiation with UVivatec^{™} Lab device in experiment JP128.

**Table 2 - Parameters**

| | *JP128* | | |
|---|---|---|---|
| *UV 254 nm (OD)* | 2.58 | | |
| *Fluency (J*/*m²)* | 200 | 300 | 400 |
| *Flow rate (l*/*h)* | 16.3 | 10.9 | 8.2 |

After the samples were subject to the different UV doses irradiation, virus inactivation was evaluated by measuring the viral titration through the TCID₅₀ assay as described in Example 1. It Was measured on the clarified harvest, *i.e*. before irradiation (Fluency 0), so as to get the initial titer of the virus fluid and after UV irradiation, so as to get the residual titer of the virus fluid after irradiation. The UV inactivating effect is illustrated by the calculation of the LVR (Log Viral Reduction) which consists in subtracting the above residual titer from the above initial titer. Results are presented in Table 3. To evaluate if a UV treatment has an impact on the virus antigenicity, the HA concentration was also determined after each UV treatment by SRD.

**Table 3 - UV effect on virus titration and HA content**

| | *JP128* | | | |
|---|---|---|---|---|
| *Fluency (J*/*m²)* | 0 | 200 | 300 | 400 |
| *log TCID₅₀*/*ml* | 8.0 | 2.6 | 2.8 | 2.8 |
| *LVR* | - | 5.4 | 5.2 | 5.2 |
| *SRD (µg HA*/*ml)* | 11.3 | 11.2 | 8.7 | 11.7 |

### - Results-Conclusions

Table 3 indicates that the HA measured by SRD was not affected by any of the tested UV dose. A decrease in the virus titer of 5.4 log was observed for a fluency as low as 200 J/m². None of the UV doses tested did provide a virus titer equal or below the quantitation assay, suggesting that residual infectivity persisted after UV irradiation.

### - SRD method used to measure HA content

Glass plates (12.4 - 10 cm) were coated with an agarose gel containing a concentration of anti-influenza HA serum that is recommended by NIBSC. After the gel had set, 72 sample wells (3 mm diameter) were punched into the agarose. 10 µl of appropriate dilutions of the reference and the sample were loaded in the wells. The plates were incubated for 24 hours at room temperature (20 to 25°C) in a moist chamber. After that, the plates were soaked overnight with NaCl solution and washed briefly in distilled water. The gel was then pressed and dried. When completely dry, the plates were stained on Coomassie Brillant Blue solution for 10 minutes and destained twice in a mixture of methanol and acetic acid until clearly defined stained zones become visible. After drying the plates, the diameter of the stained zones surrounding antigen wells was measured in two directions at righit angles. Alternatively equipment to measure the surface can be used. Dose-response curves of antigen dilutions against the surface were constructed and the results were calculated according to standard slope-ratio assay methods (Finney, D.J. (1952). Statistical Methods in Biological Assay. London: Griffin, Quoted in: Wood, JM, et al (1977). J. Biol. Standard. 5, 237-247).

### Example 3: Inactivation of influenza virus produced on MDCK cells induced by a combination of BPL and UV (MAP140 - Malaysia B strain, WiP144 - Wisconsin A strain and SOP138 - Solomon A strain)

The MDCK adherent cells were grown on microcarriers in perfusion culture mode at 36.5°C. After the growth phase, once the appropriate cell density was reached (above 6 x 10⁸ cells/ml), cells were inoculated with Influenza virus (Multiplicity of Infection of 1 x 10⁻⁵) in perfusion mode and the temperature was switched to 33°C. The virus was harvested by perfusion at day 5 post-inoculation. The perfusion harvests were clarified as described in step a) of Example 1. After clarification, the virus harvests were subject to the following inactivation treatments combining UV and BPL : two different UV doses were tested (200 and 500 J/m²) and combined with two BPL concentrations (0.05% and 0.1%) in any combination.

UV irradiation was performed as described in Example 2 with the same UVivatec^{™} Lab device and following the same manufacturer's recommendations. BPL was added overnight at 4°C. UV and BPL treatments were sequential, with UV irradiation performed before the incubation with BPL.

After each inactivation treatment, as indicated in the following Tables, the virus titration was determined according to the TCID₅₀ assay, as described in Example 1. The antigenicity of HA was monitored by measuring its content after the indicated steps through the SRD assay, as described in Example.2. Results are presented in Table 4 (SOP138), Table 5 (MAP140) and Table 6 (WiP144). HA results are presented in the form of percentages to be compared either to the control value 100% representing the total HA amount present in the starting material, *i.e.* present in the virus harvest before clarification (HA columns), or to the total HA amount present before performing the indicated step (HA recovery step columns).

**Table 4 - UV/BPL effect on virus titration and HA content**

| ***SOP138-Solomon A strain*** | *HA(%)* | *log TCID50*/*ml* | *HA recovery step (%)* |
|---|---|---|---|
| *Clarified Harvest* | 93 | 10.02 | 93 |
| *UV 200J*/*m²* | 80 | ≤ 0.8 | 86 |
| *UV 500J*/*m²* | 84 | 7.8 | 90 |
| *UV 200J*/*m² - BPL 0.05%* | 96 | ≤ 0.8 | 120 |
| *UV 200J*/*m² - BPL 0.1%* | 78 | ≤ 1.8 | 97 |
| *UV 500J*/*m² - BPL 0.05%* | 74 | ≤ 0.8 | 88 |
| *UV 500 J*/*m² - BPL 0.1%* | 68 | ≤ 1.8 | 82 |

| | | | |
|---|---|---|---|
| 1.8 and 0.8 are the limits of quantitation of the assay | | | |

**Table 5 - UV/BPL effect on virus titration and HA content**

| ***MAP140 - Malaysia B strain*** | *HA (%)* | *log TCID₅₀*/*ml* | *HA recovcery step (%)* |
|---|---|---|---|
| *Clarified Harvest* | 102 | 9.02 | 102 |
| *UV 200J*/*m²* | 76 | 8.12 | 75 |
| *UV 500J*/*m²* | 79 | 5.22 | 78 |
| *UV 200J*/*m² - BPL 0.05%* | 74 | ≤ 1.8 | 97 |
| *UV 200J*/*m² - BPL 0.1%* | 91 | ≤ 1.8 | 119 |
| *UV 500J*/*m² - BLP 0.05%* | 71 | ≤ 1.8 | 90 |
| *UV 500J*/*m² - BPL 0.1%* | 75 | ≤ 1.8 | 95 |

| | | | |
|---|---|---|---|
| 1.8 is the limit of quantitation of the assay | | | |

**Table 6 - UV/BPL effect on virus titration and HA content**

| **WiP144 - Wisconsin A strain** | *HA (%)* | *log TCID₅₀*/*ml* | *HA recovery step (%)* |
|---|---|---|---|
| *Harvest clarified* | 70 | 8.87 | 70 |
| *UV200J*/*m²* | 81 | ≤ 1.8 | 116 |
| UV*200J*/*m² - BPL 0.05%* | 74 | ≤ 0.8 | 92 |

| | | | |
|---|---|---|---|
| 1.8 and 0.8 are the limits of quantitation n of the assay | | | |

### - Results-Conclusions

A viral titer reduction has been observed after UV treatment for the 3 influenza strains tested. This viral reduction has been completed by the BPL action so as to achieve a virus titer which is equal or below the limit of quantitation. No major impact on the HA antigenic activity after a combined treatment of UV and BPL has been observed. Therefore, the combination of a physical treatment, such as UV irradiation, and a chemical treatment secures the influenza virus inactivation along the purification process and allows to achieve a more complete virus inactivation, as compared to the implementation of each step individually.

### Example 4: BPL-induced inactivation of influenza virus produced on EB66^{®} cells

4.1 EB66^{®} cells were grown in suspension in a batch mode. They were inoculated with H5N1 and the virus was harvested by collecting the cell culture medium a few days later. The viral harvest was clarified either by using a filtration train composed of three different depth filters with the following nominal porosities: 5 µm - 0.5 µm - 0.2 µm (EB66_26, EB66_28, EB66_33 and EB66_42) or by the successive combination of centrifugation at 4500 g and of a 0.2 µm filtration (EB66_29 and EB66_30). After clarification, the clarified viral harvest was concentrated 10-fold by ultrafiltration with a 750 kD hollow fiber filter and diafiltrated with a solution of PBS containing citrate 125 mM.

BPL 0.05% was added overnight at room temperature, either before the ultrafiltration step (EB66_26, EB66_28, EB66_29, EB66_30 and EB66_31) or after the ultrafiltration, *i.e.* BPL was added to the rententate obtained after the ultrafiltration (EB66_42 and EB66_33). Titration was measured according to the TCID₅₀ assay, as described in Example 1. It was measured on the clarified harvest (Table 7) or on the Ultrafiltration retentate (Table 8), so as to get the initial titer of the virus preparation before it was inactivated, and after the BPL treatment, so as to get the residual titer of the virus fluid after inactivation. The BPL inactivating effect is illustrated by the calculation of the LVR (Log Viral Reduction) which consists in subtracting the above residual titer from the above initial titer. Results are presented in Table 7 and Table 8 displaying the inactivating effect of BPL when implemented before the ultrafiltration step or after the ultrafiltration step, respectively. Virus titers are expressed as log TCID₅₀/ml. The last column represents the mean LVR based on the 5 different experiments. Also, the content of HA was measured by SRD as described in Example 2. HA results are presented in the form of percentages to be compared to the control value 100% representing the total HA amount present before adding BPL, *i.e.* present in the clarified harvest.

**Table 7 - BPL effect on virus titration, when BPL is added to viral clarified harvest**

| | *EB66_26* | *EB66_28* | *EB66_29* | *EB66_30* | *EB66_31* | *mean LVR* |
|---|---|---|---|---|---|---|
| *Clarified Harvest (log TCID₅₀*/*ml)* | 7.3 | 7.2 | 7.9 | 7.8 | 7.5 | |
| *BPL 0.05% (log TCID₅₀*/*ml)* | ≤2.2 | <1.6 | ≤1.6 | ≤1.6 | ≤1.6 | |
| *LVR* | ≥5.1 | ≥5.6 | ≥6.3 | ≥6.2 | ≥5.9 | ≥5.8 |
| *HA step recovery (%)* | 118 | 100 | 97 | 130 | 106 | |

| | | | | | | |
|---|---|---|---|---|---|---|
| 2.2 and 1.6 are the limits of quantitation of the assay. | | | | | | |

**Table 8 - BPL effect on virus titration, when BPL is added to the concentrated clarified viral harvest**

| | *EB66_42* | *EB66_33* |
|---|---|---|
| *UF retentate (log TCID₅₀*/*ml)* | 8.1 | 8.0 |
| *EPL 0.05% (log TCID₅₀*/*ml)* | ≤2.2 | 3.4 |
| *LVR* | ≥5.9 | 4.6 |

| | | |
|---|---|---|
| * UF is for Ultrafiltration. 2.2 is the limit of quantitation of the assay. | | |

### - Results-Conclusions

Table 7 indicates that the treatment of the viral clarified harvest with BPL was effective at inactivating viruses, as compared to the controls where no BPL was added. BPL 0.05% resulted in a viral titer which is equal or below the limit of quantitation of the assay and allowed achieving a LVR of about 5.8 logs, as compared to the controls where no BPL was added. Table 8 shows that the treatment of the viral clarified harvest after its has been concentrated by ultrafiltration with BPL 0.05% gives a titer, on the one hand, equal or below the limit of quantitation of the assay (see EB66_42), and, on the other hand, above the limit of quantitation (see EB66_33 showing a residual level of infectious virus, as the titer obtained is above the limit of quantitation), exhibiting a LVR ≥5.9 and of 4.6, respectively.

4.2 In a separate experiment, EB66^{®} cells were inoculated with H5N1 and the virus was harvested a few days later by collecting the cell culture medium. The viral harvest was clarified by the successive combination of a centrifugation at 8500 rpm and a 0.45 µm filtration (EB66_65 and 69). The clarified viral harvest was then concentrated around 10-fold by ultrafiltration and diafiltered with a solution of PBS (EB66_69) or a solution of PBS containing citrate 125 mM, pH 7.4. The ultrafiltration retentate was then subject to a sucrose gradient (0-55%) ultracentrifugation step as described in Example 1. The whole virion pooled fractions were then directly added with BPL 0.05%. Titration was measured according to the TCID₅₀ assay, as described in Example 1. It was measured before adding the BPL, so as to obtain the initial titer of the virus fluid before it is inactivated, and after BPL treatment, so as to get the residual titer of the virus preparation after inactivation. The BPL inactivating effect is illustrated by the calculation of the LVR. Results are presented in Table 9.

**Table 9 - BPL effect on virus titration, when BPL is added to sucrose gradient-purified whole virion fractions.**

| | *EB66_65* | *EB66_69* |
|---|---|---|
| *Before BPL 0.05% addition (log TCID₅₀*/*ml)* | 9.0 | 8.8 |
| *After BPL 0.05% treatment (log TCID₅₀*/*ml)* | ≤2.8 | ≤1.6 |
| *LVR* | ≥6.2 | ≥7.2 |

| | | |
|---|---|---|
| 2.2 is the limit of quantitation of the assay | | |

### - Results-Conclusions

Table 9 indicates that BPL 0.05% was effective at inactivating viruses, allowing to achieve a LVR of more than 6 logs. These results indicate that implementing a BPL treatment immediately after a sucrose gradient ultracentrifugation step provides good inactivation results, suggesting that residual sucrose does not negatively impact the inactivating effect of BPL. The LVR achieved in those BPL conditions is slightly higher than the ones obtained in Tables 7 and 8.

### Example 5: UV-induced inactivation of influenza virus produced on EB66^{®} cells

EB66^{®} cells were cultured and infected with H5N1 as described in Example 4. The viral harvest was clarified by a successive centrifugation at 4500 g and a filtration on a 0.2 µm filter. The viral clarified harvest was then concentrated by ultrafiltration as described in Example 4. No BPL was added in those experiments.

After ultrafiltration, the retentate was subject to a dose range of UV irradiation (100 J/m², 150 J/m² and 200 J/m²), as described in Example 2, with the same UVivatec^{™} Lab device and following the same manufacturer's recommendations. After irradiation, the virus titration was determined according to the TCID₅₀ assay, as described in Example 1. It was measured before UV irradiation in a sample collected from the ultrafiltration retentate, so as to get the initial titer of the virus fluid (and after UV irradiation, so as to get the residual titer of the virus fluidafter inactivation. The UV inactivating effect is illustrated by the calculation of the LVR (log viral reduction) which consists in subtracting the above residual titer from the above initial titer. Results are presented in Table 10. Virus titers are expressed as log TCID₅₀/ml.

**Table 10 - UV effect on virus titration, when UV irradiation implemented on the concentrated clarified harvest**

| *EB66_25* | *log TCID₅₀*/*ml* | *LVR* |
|---|---|---|
| *UF* retentate* | 7.8 | |
| *UV 100 J*/*m²* | ≤2.2 | ≥5.6 |
| *UV 150 J*/*m²* | ≤2.2 | ≥5.6 |
| *UV 200 J*/*m²* | ≤2.2 | ≥5.6 |

| | | |
|---|---|---|
| * UF is for Ultrafiltration, 2.2 is the limit of the quantitation of the assay | | |

### - Results-Conclusions

Table 10 indicates that a UV dose ranging from 100 to 200 J/m² was effective at inactivating influenza viruses produced on EB66^{®} cells, as the virus titer was equal or below the limit of quantitation of the assay.

### Example 6: BPL-and UV-induced inactivation of influenza virus produced on EB66^{®} cells

6.1 EB66^{®} cells were cultured and infected with H5N1 as described in Example 4. The viral harvest was clarified by a successive centrifugation at 10500 rpm and microfiltration on a 0.45 µm filter.

The clarified harvest was subject to (i) a BPL 0.01% treatment, (ii) a UV 20 J/m² irradiation, or (iii) a BPL 0.01% treatment and a UV 60 J/m² irradiation. BPL 0.01% was left overnight at room temperature. The UV irradiation was as described in Example 2, using the same UVivatec^{™} Lab device and following the same manufacturer's recommendations. In condition (iii), the clarified harvest was first treated with BPL 0.01% overnight at room temperature, and then, the next day, it was irradiated with a UV dose of 60 J/m². The virus titration was determined according to the TCID₅₀ assay, as described in Example 1, before adding the BPL or irradiating with UV, so as to get the initial titer of the virus fluid and after BPL treatment and/or UV irradiation, so as to get the residual titer of the virus fluid after inactivation. The BPL and/or UV inactivating effect is illustrated by the calculation of the LVR (log viral reduction) which consists in subtracting the above residual titer from the above initial titer. Results are presented in Table 11. Virus titers are expressed as log TCID₅₀/ml.

**Table 11 - BPL/UV effect on virus titration, when implemented on the clarified harvest**

| | *log TCID₅₀*/*ml* | *LVR* |
|---|---|---|
| *Clarified Harvest* | 7.9 | - |
| *BPL 0.01%* | 5.8 | 2.1 |
| *UV 20 J*/*m²* | 5.4 | 2.5 |
| *BPL 0.01% UV 60 J*/*m²* | ≤2.2 | ≥5.7 |

| | | |
|---|---|---|
| 2.2 is the limit of the quantitation of the assay | | |

### - Results-Conclusions

Table 11 indicates that BPL at a 0.01% concentration partially inactivates Flu-containing clarified harvest, as residual infectivity was measured (LVR is of 2.1 log), while Table 9 provided results where BPL 0.05% allowed to achieve a more complete virus inactivation, as the titer obtained was equal or below the limit of quantitation of the assay. However, when combining the suboptimal BPL 0.01% concentration with a UV 60 J/m² irradiation step, then the titer obtained was equal or below the limit of quantitation of the assay, suggesting that the combination of a physical treatment, such as UV, and a' chemical treatment, such as BPL, secures the influenza virus inactivation along the purification process, allowing to achieve a more complete virus inactivation, while limiting the amount of BPL which needs to be used for that effect.

6.2 In a separate experiment, EB66^{®} cells were cultured and infected with H5N1 as described in Example 4. The viral harvest was collected and clarified as described in Example 4. BPL 0.05% was added overnight at room temperature to the clarified viral harvest. The BPL-treated viral harvest was, then, concentrated 10-fold by ultrafiltration with a 750 kD hollow fiber filter and diafiltrated with a solution of PBS containing citrate 125 mM. After concentration, the BPL-treated viral harvest was irradiated with a 200 J/m² UV dose. Virus titration was measured before adding BPL in a sample collected from the indicated clarified harvest, so as to get the initial titer of the virus fluid, after BPL addition, and after UV irradiation, so as to get the residual titer of the virus preparation after inactivation. The inactivating effect of BPL and BPL/UV is illustrated by the calculation of the LVR (Log Viral Reduction) which consists in subtracting the above residual titer from the above initial titer. Results are presented in Table 13. Virus titers are expressed as log TCID₅₀/ml.

**Table 12 - BPL/UV effect on virus titration**

| | *EB66_30* | | *EB66_31* | |
|---|---|---|---|---|
| | *log TCID₅₀*/*ml* | *LVR* | *log TCID₅₀*/*ml* | *LVR* |
| *Clarified Harvest* | 7.8 | - | 7.5 | - |
| *BPL 0.05%* | ≤1.6 | ≥6.2 | ≤1.6 | ≥5.9 |
| *UV 200 J*/*m²* | ≤1.6 | ≥6.2 | ≤1.6 | ≥5.9 |

| | | | | |
|---|---|---|---|---|
| 1.6 is the limit of quantitation of the assay | | | | |

### - Results-Conclusions

BPL addition to the virus clarified harvest and subsequent UV irradiation are effective at inactivating influenza virus, as such a combined treatment results in a virus titer which is equal or below the limit of quantitation of the assay.

### Example 7: Triton X-100-induced inactivation of influenza virus produced on EB66^{®} cells

EB66^{®} cells were cultured and infected with H5N1 as described in Example 4. The viral harvest was collected, clarified and concentrated by ultrafiltration as described in Example 4. No BPL was added, nor was any UV irradiation implemented during the process. The concentrated viral harvest was then subject to a sucrose gradient (0-55%) ultracentrifugation, wherein virus and contaminants migrated into the gradient until reaching their respective density. Once all the retentate was loaded onto the gradient, a banding time of 60 minutes allowed most of the virus to reach its density within the gradient. The viral particles were concentrated within a few fractions. The product fractions were in PBS pH 7.4 containing 125 mM citrate and sucrose. The purified whole virion was pooled from the percentage of sucrose raging from approximately 30 to 48%..This range has been determined on the basis of profiles from SDS-PAGE and from Western Blot analysis using anti-HA and anti-MDCK antibodies. The whole virus pooled fractions were, then, concentrated by ultrafiltration with a 750 kD hollow fiber filter and diafiltrated with a solution of PBS.

Triton X-100 2% was added at room temperature to the whole virus-containing fractions collected and pooled after sucrose gradient centrifugation in order to split the virus. Virus titration was measured before adding Triton X-100, so as to get the initial titer of the virus fluid and at different time-points (as indicated in Table 12) after Triton X-100 addition, so as to get the residual titer of the virus preparation after splitting. The Triton X-100 inactivating effect is illustrated by the calculation of the LVR (Log Viral Reduction) which consists in subtracting the above residual titer from the above initial titer. Results are presented in Table 13. Virus titers are expressed as log TCID₅₀/ml.

**Table 13 - Triton X-100 effect on virus titration**

| | *EB66_33* | | *EB66_39* | |
|---|---|---|---|---|
| *Triton X-100 2%* | *log TCID₅₀*/*ml* | *LVR* | *log TCID₅₀*/*ml* | *LVR* |
| *UF retentate* | 8.4 | | 8.8 | |
| *t = 30 min* | 5.2 | 3.2 | | |
| *t = 2 h* | | | 5.0 | 3.8 |

| | | | | |
|---|---|---|---|---|
| * UF is for Ultrafiltration | | | | |

### - Results-Conclusions

Triton X-100 2% is effective at inactivating influenza virus after a treatment time as short as 30 minutes. A LVR of 3.2 logs and 3.8 logs was observed after 30 minutes and 2 hours of treatment, respectively.

### Example 8: Cumulative effect of BPL-, UV- and Triton X-100-induced inactivation of influenza virus produced on EB66 cells

In order to evaluate the cumulative effect of implementing BPL, UV and Triton X-100 steps within the same process, LVR obtained in the Examples 4, 5 and 7 for the individual steps, BPL, UV and Triton, respectively, were added up. Results are presented in Table 14.

**Table 14 - Cumulative effect of BPL, UV and Triton X-100 on virus titration**

| | *LVR* |
|---|---|
| *BPL 0.05%* | ≥5.8* |
| *UV 200 J*/*m²* | ≥5.6** |
| *Triton X-100 2%* | 3.8*** |
| *Cumulative LVR* | ≥15.2 |

| | |
|---|---|
| * This value corresponds to the mean LVR presented in Table 7, where BPL 0.05% was added after clarification ** This value corresponds to the value presented in Table 10, where UV was implemented on the UF retentate *** This value corresponds to the value presented in Table 13, where Triton-X100 was added for 2 hours | |

### - Results-Conclusions

The combination of 3 individual steps of inactivation within the same process (BPL; UV and Triton X-100) results in a LVR higher than 15.2

### Example 9: Cumulative effect of BPL- and UV-induced inactivation of adventitious viruses

The use of animal cells for influenza production, in particular, for inclusion in a vaccine, involves culturing the cells under conditions that are suited to viral growth and replication. Therefore, these conditions increase the risk that pathogens other than influenza virus may grow in the cell culture and, thereby leading to potential contamination of the final vaccine product with adventitious viruses. The virus preparation obtained according to the method of the invention was tested against 4 model adventitious viruses: Porcine Pseudorabies Virus (PRV), Murine Leukaemia Virus (MuLV), Porcine Parvovirus (PPV) and Hepatitis A Virus (HAV). Before proceeding to the indicated inactivation step (UV or BPL), influenza virus fluids were spiked with the indicated adventitious virus.

9.1 Influenza virus was produced in MDCK cells as described in Example 1. The virus fluid was spiked separately with MuLV and PPV. BPL 0.05% and BPL 0.1 % were added overnight at room temperature to each spiked influenza virus preparation. Titration was measured according to the TCID₅₀ assay, as described in Example 1. It was measured before adding BPL, so as to get the initial titer of the virus fluid before it was inactivated, and after the BPL treatment, so as to get the residual titer of the virus fluid after inactivation. The BPL inactivating effect is illustrated by the calculation of the LVR (Log Viral Reduction) which consists in subtracting the above residual titer from the above initial titer. Independently, influenza virus preparation separately spiked with MuLV and PPV was also irradiated with a 200 J/m² UV dose. Titration was measured before irradiation with UV, so as to get the initial titer of the virus preparation before it was inactivated and after the UV irradiation, so as to get the residual titer of the virus preparation after inactivation. The BPL and UV inactivating effect is illustrated by the calculation of the LVR (Log Viral Reduction) which consists in subtracting the above residual titer from the above initial titer. Results are presented in Table 15. Virus titers are expressed as log TCID₅₀/ml.

**Table 15 - Effect of BPL and UV on MuLV and PPV virus titration**

| | *MuLV* | | *PPV* | |
|---|---|---|---|---|
| | *log TCID₅₀*/*ml* | *LVR* | *log TCID₅₀*/*ml* | ***LVR*** |
| No BPL | 6.0 | | 8.1 | |
| BPL 0.05% | 3.3 | 2.7 | 3.9 | 4.2 |
| No BPL | 6.5 | | 8.1 | |
| BPL 0.1% | 3.7 | 2.8 | 2.4 | 5.7 |
| No UV | 6.1 | | 8.3 | |
| UV 200 J/m² | 3.5 | 2.6 | 0.1 | 8.2 |

### - Results-Conclusions

BPL treatment resulted in a reduction in the virus titer with respect to both MuLV and PPV at the two concentrations tested. UV treatment also resulted in a reduction in the virus titer of both MuLV and PPV, with a particularly strong effect on PPV, as the LVR observed was 8.2 logs. The combination treatment of BPL and UV secures the purification process of influenza virus produced in cell culture from contamination with adventitious viruses, if any, by inactivating said viruses.

9.2 Influenza virus produced in EB66^{®} cells as described in Example 4 was separately spiked with PPV, PRV, HAV, and MuLV. After spiking, each influenza virus fluid was inactivated either by BPL 0.05% added overnight at room temperature or by UV irradiation with a dose of 200 J/m², as indicated in Table 16. Virus titration was measured according to the TCID₅₀ assay, as described in Example 1. It was measured before adding BPL or irradiating with UV, so as to get the initial titer of the virus fluid before it was inactivated, and after the BPL treatment or after the UV irradiation, so as to get the residual titer of the virus fluid after inactivation. The BPL and UV inactivating effect is illustrated by the calculation of the LVR (Log Viral Reduction) which consists in subtracting the above residual titer from the above initial titer. Results are presented in Tables 16 and 17. Virus titers are expressed as log TCID₅₀/ml.

**Table 16 - Effect of BPL and UV on MuLV and PPV virus titration**

| | *MuLV* | | *PPV* | |
|---|---|---|---|---|
| | *log TCID₅₀*/*ml* | *LVR* | *log TCID₅₀*/*ml* | *LVR* |
| No BPL | 5.8 | | 8.1 | |
| BPL 0.05 | 4.2 | 1.6 | 6.0 | 2.1 |
| No UV | | | 8.2 | |
| UV 200 J/m² | | ≤1 | 1.4 | 6.8 |

**Table 17 - Effect of BPL and UV on HAV and PRV virus titration**

| | *HAV* | | *PRV* | |
|---|---|---|---|---|
| | *log TCID₅₀*/*ml* | *LVR* | *log TCID₅₀*/*ml* | *LVR* |
| No BPL | 5.3 | | 6.0 | |
| BPL 0.05% | 1.5 | 3.8 | 2.6 | 3.4 |
| No UV | 5.4 | | | |
| UV 200 J/m² | ≤1.4 | ≥4 | | 3.67 |

### - Results-Conclusions

BPL treatment resulted in a reduction in the virus titer with respect to all tested viruses, MuLV, PPV, HAV and PRV. UV treatment resulted in a reduction in the virus titer of PRV, PPV and HAV with a particularly strong effect on PPV, as the LVR observed was 6.8 logs. Therefore, the combination treatment of BPL and UV secures the purification process of influenza virus produced in cell culture from contamination with adventitious viruses, if any, by inactivating said viruses.

## Claims

1. A method for inactivating an influenza virus propagated in cell culture and inactivating contaminating adventitious viruses, comprising at least the following steps:
(a) treating an influenza virus-containing fluid with beta-propiolactone, and
(b) irradiating the influenza virus-containing fluid with UV light.

2. The method according to claim 1, wherein steps (a) and (b) are performed sequentially, in any order.

3. The method according to claims 1 and 2, wherein beta-propiolactone is used at a concentration ranging from 0.01% to 0.1%, or from 0.03% to 0.8%, or at a concentration of 0.05%.

4. The method according to any of preceding claims, wherein the UV dose ranges from 50 to 500J/m², from 100 to 400 J/m², or is 200 J/m², or is 100 J/m².

5. The method according to any of claims 1 to 4, further comprising at least one virus purification step selected from the group consisting of: clarification, ultrafiltration, nucleic acid degradation, ultracentrifugation and chromatography.

6. The method according to claim 5, wherein the virus is purified by clarification.

7. The method according to claim 6, wherein steps (a) and (b) are implemented after clarification.

8. The method according to claims 5 and 6, wherein the virus is purified by sucrose gradient ultracentrifugation.

9. The method according to claim 8, wherein steps (a) and (b) are implemented after the sucrose gradient ultracentrifugation.

10. The method according to claim 9, wherein step (a) is implemented before step (b).

11. The method according to claims 1 to 10, further comprising a splitting step.

12. The method according to claims 1 to 11, wherein the influenza virus is propagated in EB66^{®} cells.

13. The method according to claims 1 to 12, wherein the adventitious viruses are selected from: Murine Leukaemia virus, Hepatitis A virus, Porcine Parvovirus, Porcine Pseudorabies virus, or any combination thereof.

14. A method for the preparation of a vaccine comprising an influenza virus propagated in cell culture comprising the following steps:
(a) treating an influenza virus-containing and contaminating adventitious viruses containing fluid with beta-propiolactone,
(b) irradiating the influenza virus-containing and contaminating adventitious viruses containing fluid with UV light, and
(c) formulating the beta-propiolactone-treated and UV-irradiated influenza virus into a vaccine.

## Patentansprüche

1. Verfahren zum Inaktivieren eines Influenzavirus, der in einer Zellkultur vermehrt wurde, und Inaktivieren kontaminierender adventiver Viren, das mindestens die folgenden Schritte umfasst:
(a) Behandeln einer Flüssigkeit, die einen Influenzavirus enthält, mit Beta-Propiolacton und
(b) Bestrahlen der Flüssigkeit, die einen Influenzavirus enthält, mit UV-Licht.

2. Verfahren nach Anspruch 1, wobei Schritte (a) und (b) nacheinander in beliebiger Reihenfolge durchgeführt werden.

3. Verfahren nach den Ansprüchen 1 und 2, wobei Beta-Propiolacton in einer Konzentration im Bereich von 0,01% bis 0,1% oder von 0,03% bis 0,8%, oder in einer Konzentration von 0,05% verwendet wird.

4. Verfahren nach einem der vorangehenden Ansprüche, wobei die UV-Dosis im Bereich von 50 bis 500 J/m², von 100 bis 400 J/m² liegt oder 200 J/m² ist oder 100 J/m² ist.

5. Verfahren nach einem der Ansprüche 1 bis 4, des Weiteren umfassend mindestens einen Virus-Reinigungsschritt, der ausgewählt ist aus der Gruppe bestehend aus: Klärung, Ultrafiltration, Nucleinsäure-Abbau, Ultrazentrifugierung und Chromatographie.

6. Verfahren nach Anspruch 5, wobei der Virus durch Klärung gereinigt ist.

7. Verfahren nach Anspruch 6, wobei Schritte (a) und (b) nach der Klärung durchgeführt werden.

8. Verfahren nach den Ansprüchen 5 und 6, wobei der Virus durch Saccharose-Gradienten-Ultrazentrifugierung gereinigt wird.

9. Verfahren nach Anspruch 8, wobei Schritte (a) und (b) nach der Saccharose-Gradienten-Ultrazentrifugierung durchgeführt werden.

10. Verfahren nach Anspruch 9, wobei Schritt (a) vor Schritt (b) durchgeführt wird.

11. Verfahren nach den Ansprüchen 1 bis 10, des Weiteren umfassend einen Spaltungsschritt.

12. Verfahren nach den Ansprüchen 1 bis 11, wobei der Influenzavirus in EB66^{®}-Zellen vermehrt wird.

13. Verfahren nach den Ansprüchen 1 bis 12, wobei die adventive Viren sind ausgewählt aus: murinem Leukämievirus, Hepatitis-A-Virus, porcinem Parvovirus, porcinem Pseudorabies-Virus oder einer Kombination davon.

14. Verfahren zur Herstellung eines Impfstoffes, umfassend einen Influenzavirus, der in einer Zellkultur vermehrt wurde, das die folgenden Schritte umfasst:
(a) Behandeln einer Flüssigkeit, die einen Influenzavirus und kontaminierende adventive Viren enthält, mit Beta-Propiolacton,
(b) Bestrahlen der Flüssigkeit, die einen Influenzavirus und kontaminierende adventive Viren enthält, mit UV-Licht, und
(c) Formulieren des Influenzavirus, das mit Beta-Propiolacton behandelt und mit UV bestrahlt wurde, als Impfstoff.

## Revendications

1. Procédé d'inactivation d'un virus de la grippe propagé dans une culture cellulaire et d'inactivation de virus adventices contaminants, comprenant au moins les étapes suivantes :
(a) le traitement d'un liquide contenant le virus de la grippe avec de la bêta-propiolactone, et
(b) l'irradiation du liquide contenant le virus de la grippe avec une lumière UV.

2. Procédé selon la revendication 1, dans lequel les étapes (a) et (b) sont effectuées séquentiellement, dans un ordre quelconque.

3. Procédé selon les revendications 1 et 2, dans lequel la bêta-propiolactone est utilisée à une concentration située dans la plage de 0,01 % à 0,1 % ou de 0,03 % à 0,8 %, ou à une concentration de 0,05 %.

4. Procédé selon l'une quelconque des revendications précédentes, où la dose d'UV se situe dans la plage de 50 à 500 j /m², de 100 à 400 J/m² ou est de 200 J/m², ou est de 100 J/m².

5. Procédé selon l'une quelconque des revendications 1 à 4, comprenant en outre au moins une étape de purification du virus choisie dans le groupe constitué de : clarification, ultrafiltration, dégradation des acides nucléiques, ultracentrifugation et chromatographie.

6. Procédé selon la revendication 5, dans lequel le virus est purifié par clarification.

7. Procédé selon la revendication 6, dans lequel les étapes (a) et (b) sont mises en oeuvre après la clarification.

8. Procédé selon les revendications 5 et 6, dans lequel le virus est purifié par ultracentrifugation sur gradient de saccharose.

9. Procédé selon la revendication 8, dans lequel les étapes (a) et (b) sont mises en oeuvre après l'ultracentrifugation sur gradient de saccharose.

10. Procédé selon la revendication 9, dans lequel l'étape (a) est mise en oeuvre avant l'étape (b).

11. Procédé selon les revendications 1 à 10, comprenant en outre une étape de fragmentation.

12. Procédé selon les revendications 1 à 11, dans lequel le virus de la grippe est propagé dans des cellules EB66®.

13. Procédé selon les revendications 1 à 12, dans lequel les virus adventices sont choisis parmi : le virus de la leucémie murine, le virus de l'hépatite A, le parvovirus porcin, le virus de la pseudorage porcine, ou l'une quelconque de leurs combinaisons.

14. Procédé de préparation d'un vaccin comprenant un virus de la grippe propagé dans une culture cellulaire comprenant les étapes suivantes :
(a) le traitement d'un liquide contenant le virus de la grippe et contenant des virus adventices contaminants avec de la bêta-propiolactone,
(b) l'irradiation du liquide contenant le virus de la grippe et contenant des virus adventices contaminants avec une lumière UV, et
(c) la formulation du virus de la grippe traité par la bêta-propiolactone et irradié aux UV dans un vaccin.
